# EUROPEAN PATENT APPLICATION

(11) **EP 3 195 822 A1**
(43) Date of publication of application: **26.07.2017**
(21) Application number: 17152609.8
(22) Date of filing: 23.01.2017
(51) Int. Cl.: A61B 18/14, A61B 17/10, A61B 34/37, A61B 17/128, A61B 18/00

(54) **DEVICES FOR TISSUE SEALING AND MECHANICAL CLIPPING**

(30) Priority: 23.01.2016 US 201662286352 P; 19.01.2017 US 201715409593
(71) Applicant: Covidien LP, Mansfield, MA 02048 (US)
(72) Inventor: Kerr, Duane E., Loveland, CO 80537 (US); Allen, James D., Broomfield, CO 80020 (US)
(74) Representative: Maschio & Soames IP Ltd

(57) **Abstract**

An end effector assembly includes a first jaw member having a first tissue-contacting surface, a second jaw member coupled to the first jaw member and having a second tissue-contacting surface, and one or more clips supported by the first and second jaw members. The first and second tissue-contacting surfaces conduct electrical energy to seal tissue disposed between the first and second jaw members. The first and second jaw members deform the one or more clips such that the one or more clips reinforce a tissue seal formed between the first and second jaw members.

## Description

### CROSS REFERENCE TO RELATED APPLICATION

The present application claims the benefit of and priority to U.S. Provisional Application Serial No. 62/286,352, filed on January 23, 2016, the entire contents of which are incorporated herein by reference.

### TECHNICAL FIELD

The present disclosure relates to surgical instruments, and more particularly, to surgical instruments for sealing and clipping tissue.

### BACKGROUND

Surgical instruments such as energy-based devices are typically used in conjunction with energy sources (external energy sources or portable energy sources incorporated into the instruments themselves) to apply and control the application of energy to tissue to thermally treat tissue (e.g., heat) to achieve a desired tissue effect. Electrosurgical forceps, for example, utilize both the mechanical clamping action of jaw members thereof and the energy provided by the energy source to heat tissue grasped between the jaw members for achieving a desired tissue effect such as sealing. Typically, after grasped tissue is sealed, a clinician advances a blade through the electrosurgical forceps to sever the sealed tissue while the sealed tissue is disposed between the jaw members.

While typical energy-based tissue seals created with these surgical instruments may provide adequate sealing, it would be advantageous to provide further assurances of the effectiveness of these energy-based tissue seals.

### SUMMARY

According to one aspect of the present disclosure, an end effector assembly is provided. The end effector assembly includes a first jaw member having a first tissue-contacting surface, a second jaw member coupled to the first jaw member and having a second tissue-contacting surface, and one or more clips supported by the first and second jaw members. The first and second tissue-contacting surfaces may be configured to conduct electrical energy to seal tissue disposed between the first and second jaw members. The first and second jaw members may be configured to deform the one or more clips such that the one or more clips reinforce a tissue seal formed between the first and second jaw members.

In some embodiments, the end effector assembly may further comprise a knife assembly including a knife blade configured to advance along one or both of the first and second jaw members to sever tissue sealed and clipped between the first and second jaw members. One or both of the first and second jaw members may include a knife channel defined therein that extends along a length thereof and is configured to receive the knife blade of the knife assembly during translation thereof. The first tissue-contacting surface may include a first section on a first side of the knife channel and a second section on a second side of the knife channel. The first and second sections of the first tissue-contacting surface may act as counterpart electrodes that conduct electrical energy laterally across the knife channel. The second tissue-contacting surface may include a first section on the first side of the knife channel and a second section on the second side of the knife channel. The first and second sections of the second tissue-contacting surface may act as counterpart electrodes that conduct electrical energy laterally across the knife channel.

In embodiments, the one or more clips may include two clips supported between the first and second jaw members. The first and second jaw members may be positionable between open and closed positions to deform the two clips between the first and second jaw members.

In some embodiments, one or both of the first and second jaw members define one or more clip slots configured to support the one or more clips between the first and second jaw members. The one or more clip slots include a pair of clip slots defined on opposite sides of a knife channel defined in one or both of the first and second jaw members. The knife channel may extend along a length of one or more of the first and second jaw members. The one or more clips may include a pair of clips. The pair of clip slots may be configured to support the pair of clips between the first and second jaw members.

In embodiments, the first and second tissue-contacting surfaces may include seal plates that extend between the pair of clip slots. The pair of clip slots may be positioned to electrically isolate the seal plates. The seal plate of the first tissue-contacting surface may act as a first electrode and the seal plate of the second tissue-contacting surface may act as a second electrode. The first and second electrodes may conduct electrical energy between the pair of clip slots to seal tissue disposed between the pair of clip slots.

According to another aspect, the present disclosure is directed to a forceps including a handle, an elongated shaft extending from the handle, and an end effector assembly secured to a distal end of the elongated shaft.

Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description given below, serve to explain the principles of the disclosure, wherein:
FIG. 1 is a perspective view of a surgical instrument in accordance with the principles of the present disclosure;
FIG. 2A is an enlarged, side, perspective view of a distal portion of the surgical instrument of FIG. 1 shown in an open position;
FIG. 2B is an enlarged, side, perspective view of the distal portion of the surgical instrument of FIG. 1 shown in a closed position;
FIGS. 3A-3C are progressive views illustrating the surgical instrument of FIG. 1 performing a surgical procedure on tissue in accordance with the principles of the present disclosure;
FIG. 4 is a cross-sectional view of another embodiment a surgical instrument in accordance with the principles of the present disclosure;
FIGS. 5A-5C progressive views of another embodiment of a distal portion of the surgical instrument of FIG. 1 performing a surgical procedure on tissue in accordance with the principles of the present disclosure; and
FIG. 6 is a schematic illustration of a medical work station and operating console in accordance with the present disclosure.

### DETAILED DESCRIPTION

Particular embodiments of the present disclosure are described hereinbelow with reference to the accompanying drawings in which like reference numerals designate identical or corresponding elements in each of the several views. As used herein, the term "distal" refers to that portion of the system, device, and/or component(s) thereof, which is farther from the user, while the term "proximal" refers to that portion of the system, device, and/or component(s) thereof, which is closer to the user. In the following description, well-known functions or constructions are not described in detail to avoid obscuring the present disclosure in unnecessary detail.

Surgical systems in accordance with the present disclosure can include endoscopic and/or open surgical instruments such as forceps devices, ultrasonic dissection devices, and/or any other suitable surgical devices. Obviously, different electrical and mechanical connections and considerations apply to each particular type of device; however, the aspects and features of the present disclosure remain generally consistent regardless of the particular device used. For a detailed description of the construction and operation of exemplary surgical devices, reference may be made to U.S. Patent Application Publication No. 2013/0255063, U.S. Patent Application Publication No. 2012/0083786 and/or U.S. Patent No. 8,444,664, the entirety of each of which is incorporated by reference herein.

In the interest of brevity, surgical systems of the present disclosure will only be described herein in connection with an endoscopic surgical forceps.

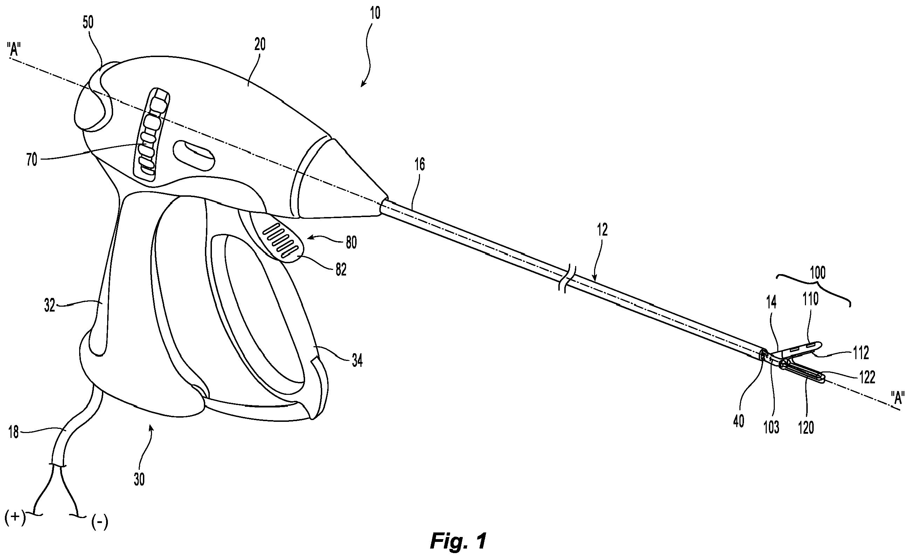

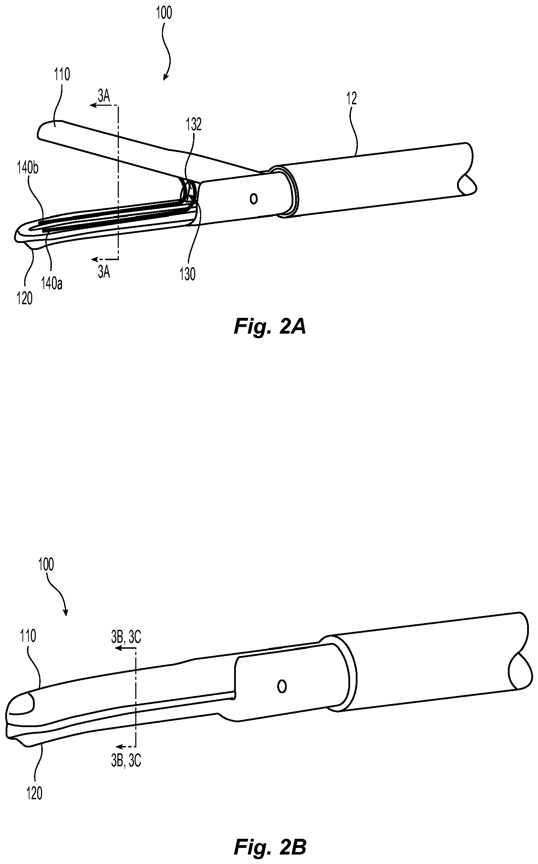

Turning now to FIGS. 1, 2A, and 2B, an electrosurgical endoscopic forceps 10 is provided. Forceps 10 has a longitudinal axis "A-A" defined therethrough, a housing 20, a handle assembly 30, a rotating assembly 70, a trigger assembly 80 and an end effector assembly 100 including first and second jaw members 110 and 120. The forceps 10 further includes a shaft 12 having a distal end 14 configured to mechanically engage the end effector assembly 100 and a proximal end 16 that mechanically engages the housing 20. The forceps 10 also includes an electrosurgical cable 18 that connects the forceps 10 to a generator (not shown) or other suitable power source. Alternately, the forceps 10 may be configured as a battery powered instrument. A cable 18, which may include one or more wires, extends through the shaft 12 to provide electrical energy to one or both of the jaw members 110, 120 of end effector assembly 100 as described in greater detail below. The handle assembly 30 supports an electrical switch 50 operatively coupled to the cable 18 to provide electrical energy from the electrosurgical energy source (e.g., generator or battery) to one or both of the jaw members 110, 120 upon actuation of the electrical switch 50.

The end effector assembly 100 is designed as a unilateral assembly, e.g., where the second jaw member 120 is fixed relative to the shaft 12 and the first jaw member 110 is moveable about a pivot 103 relative to the shaft 12 and the second jaw member 120. The handle assembly 30 includes a stationary handle 32 and a movable handle 34 that is operatively coupled to a drive mechanism 40. The movable handle 34 is movable relative to the stationary handle 32 to cause the drive mechanism 40 to pivot the first jaw member 110 about the pivot 103. In some embodiments, the end effector assembly 100 may alternatively be configured as a bilateral assembly, e.g., where both the first and second jaw members 110, 120 are moveable about the pivot 103 relative to one another and to the shaft 12. Other than jaw member 110 being movable and jaw member 120 being stationary, jaw members 110 and 120 are generally identical to one another.

In certain embodiments, the first and second jaw members 110, 120 are configured to move in parallel relation to one another between an open (unapproximated) position and a closed (approximated) position. In some embodiments, a knife assembly 130 is disposed within the shaft 12 and opposing knife channels 112, 122 are defined within the first and second jaw members 110, 120, respectively, to permit reciprocation of a knife blade 132 (FIG. 2A) of the knife assembly 130 therethrough, e.g., via activation of a trigger 82 of the trigger assembly 80. Knife channels 112, 122 are positioned in vertical registration with one another and cooperate as a unit when jaw members 110, 120 are disposed in a closed (approximated) position to enable reciprocation of the knife blade 132 through the jaw members 110, 120.

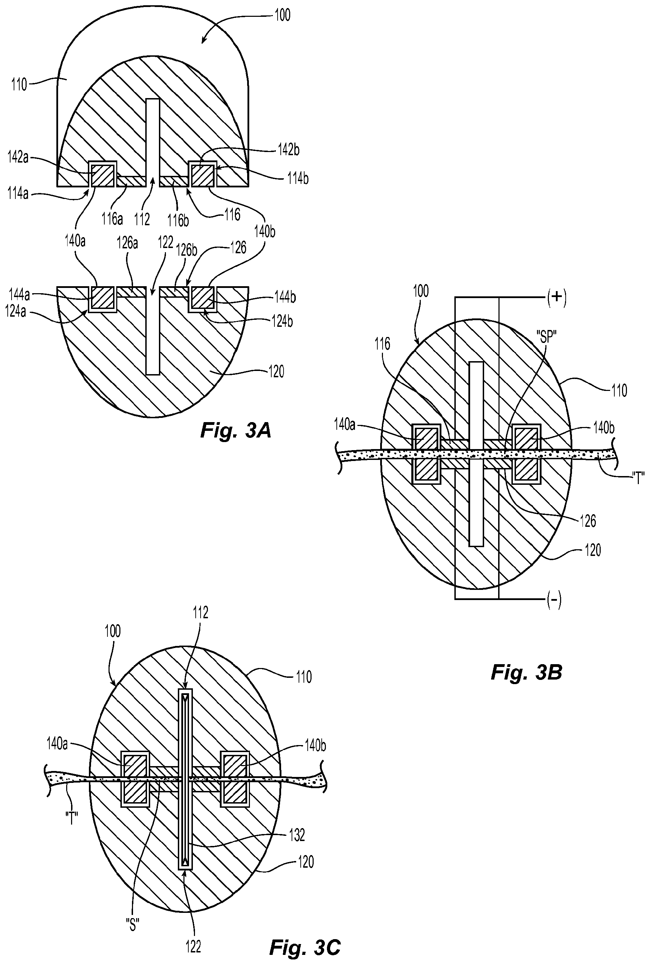

Turning now to FIGS. 3A-3C, the first jaw member 110 has clip slots 114a, 114b defined therein on opposite sides of the knife channel 112. Similarly, the second jaw member 120 has clip slots 124a, 124b defined therein on opposite sides of the knife channel 122. The clip slots 114a, 124a of respective first and second jaw members 110, 120 are disposed in vertical registration relative to one another and support a clip 140a between the first and second jaw members 110, 120. The clip slot 114a supports a first leg 142a of the clip 140a and the clip slot 124a supports a second leg 144a of the clip 140a. The clip slots 114b, 124b of respective first and second jaw members 110, 120 are disposed in vertical registration relative to one another and support a clip 140b between the first and second jaw members 110, 120. The clip slot 114b supports a first leg 142b of the clip 140b and the clip slot 124b supports a second leg 144b of the clip 140b.

The first jaw member 110 includes a tissue-contacting surface 116 supported between the clip slots 114a, 114b, and which may be in the form of a seal plate. The clips slots 114a, 114b may be positioned to electrically isolate tissue-contacting surface 116 between the clip slots 114a, 114b. The tissue-contacting surface 116 includes a first section 116a and a second section 116b. The first and second sections 116a, 116b of the tissue-contacting surface 116 are supported on opposite sides of the knife channel 112 and are operatively coupled to an electrosurgical energy source (e.g., via cable 18 shown in FIG. 1). The first and second sections 116a, 116b are configured to act collectively as a first electrode.

The second jaw member 120 includes a tissue-contacting surface 126 supported between the clip slots 124a, 124b, and which may be in the form of a seal plate. The clips slots 124a, 124b may be positioned to electrically isolate tissue-contacting surface 126 between the clip slots 124a, 124b. The tissue-contacting surface 126 includes a first section 126a and a second section 126b. The first and second sections 126a, 126b of the tissue-contacting surface 126 are supported on opposite sides of the knife channel 122 and are operatively coupled to an electrosurgical energy source (e.g., via cable 18 shown in FIG. 1). The first and second sections 126a, 126b are configured to act collectively as a second electrode such that the tissue-contacting surfaces 116, 126 of first and second jaw members 110, 120 are configured to conduct electrical energy vertically between one another (e.g., through tissue) to effectuate tissue sealing as described in greater detail below.

Although the tissue-contacting surfaces 116, 126 of the first and second jaw members 110, 120 are illustrated extending along only a portion of a width of respective first and second jaw members 110, 120 (e.g., a central portion), the tissue-contacting surfaces 116, 126, in some embodiments, may extend along any portion (e.g., continuous and/or discontinuous portions) and/or an entirety of the width and/or the length of respective first and second jaw members 110, 120.

In use, the movable handle 34 is actuated to enable the first and second jaw members 110, 120 to move from the open position (FIG. 3A) to the closed position (FIG. 3B) to thereby grasp tissue "T" disposed between the first and second jaw members 110, 120 while deforming the clips 140a, 140b onto the tissue "T" disposed between the first and second jaw members 110, 120. As the first and second jaw members 110, 120 move toward the closed position, the first and second jaw members 110, 120 approximate the first and second legs 142a, 144a of the clip 140a about the tissue "T" on a first side of the tissue-contacting surfaces 116, 126 of the first and second jaw members 110, 120 while simultaneously approximating the first and second legs 142b, 144b of the clip 140b about the tissue "T on a second side of the tissue-contacting surfaces 116, 126 of the first and second jaw members 110, 120 to capture a sealing portion "SP" of the tissue "T" to be sealed between clips 140a, 140b.

As seen in FIGS. 3B and 3C, a clinician can then actuate the electrical switch 50 to conduct electrosurgical energy between the tissue-contacting surfaces 116, 126 (via tissue), which act as counterpart electrodes (e.g., anode-cathode), to seal the sealing portion "SP" of the tissue "T" and create sealed tissue "S." With the sealed tissue "S" supported between the clips 140a, 140b, the trigger 82 of the trigger assembly 80 (see FIG. 1) can be actuated to advance the knife blade 132 of the knife assembly 130 through the knife channels 112, 122 to sever the sealed tissue "S." The clips 140a, 140b reinforce the sealed tissue "S" to provide additional closure and further ensure that the sealed tissue "S" remains sealed, further reducing risk of undesirable bleeding that may result from inadequate tissue sealing.

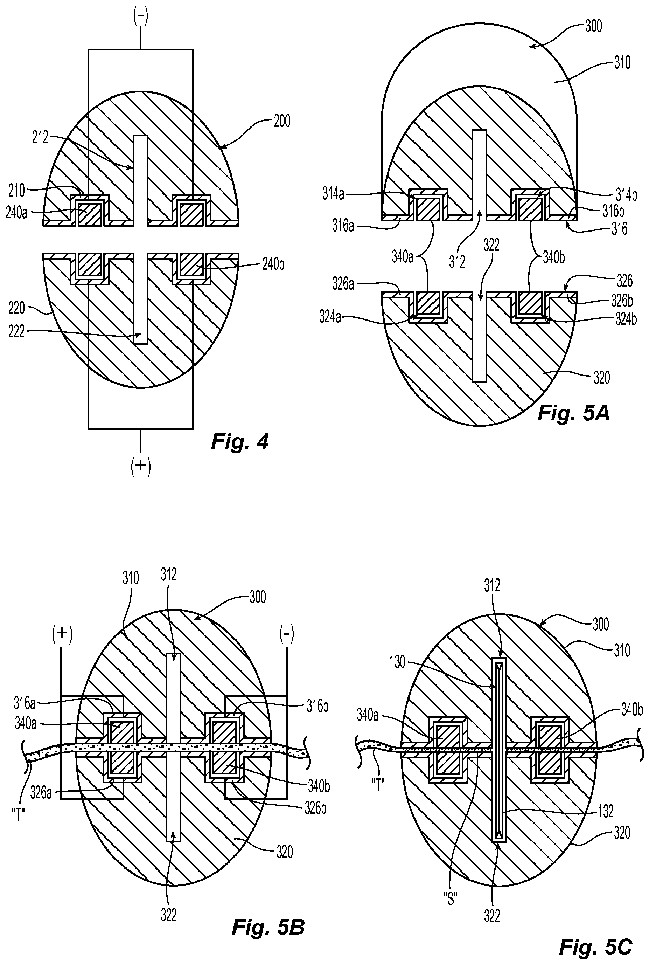

As illustrated in FIG. 4, any of the presently described end effectors, such as, end effector 200, can include first and second jaw members 210, 220 having tissue-contacting surfaces 216, 226 that extend along the width (and/or length) of respective first and second jaw members 210, 220 on both sides of each respective clip 240a, 240b to elongate a length of sealed tissue. The tissue-contacting surfaces 216, 226 may be in the form of seal plates. Also illustrated in FIG. 4, seal plates (e.g., seal plates 216, 226) of any of the presently described end effectors can be configured to conduct electrosurgical energy in any suitable direction between the first and second jaw members. For example, as seen in FIG. 4, the first jaw member 210 is negatively charged and the second jaw member 220 is positively charged; however, in an alternate embodiment, the second jaw member 220 may be negatively charged and the first jaw member 210 may be positively charged. In some embodiments, the first and/or second jaw members 210, 220 may be respectively charged so that electrosurgical energy may be conducted diagonally across the first and second jaw members 210, 220.

Turning now to FIGS. 5A-5C, another embodiment of an end effector is provided, and which is generally referred to as end effector 300. The end effector 300 includes first and second jaw members 310, 320 having tissue-contacting surfaces 316, 326, respectively, that extend along the width (and/or length) of respective first and second jaw members 310, 320, and which may be in the form of seal plates.

The tissue-contacting surface 316 of the first jaw member 310 includes a first section 316a supported by a first side of a knife channel 312 of the first jaw member 310 and a second section 316b supported by a second side of the knife channel 312. The first section 316a of the tissue-contacting surface 316 defines a clip slot 314a therein and the second section 316b of the tissue-contacting surface 316 defines a clip slot 314b therein.

The tissue-contacting surface 326 of the second jaw member 320 includes a first section 326a supported on a first side of a knife channel 322 of the second jaw member 320 and a second section 326b supported on a second side of the knife channel 322. The first section 326a of the tissue-contacting surface 326 defines a clip slot 324a therein and a second section 326b of the tissue-contacting surface 326 defines a clip slot 324b therein.

The clip slot 314a of the first jaw member 310 is in vertical registration with the clip slot 324a of the second jaw member 320 to support the clip 340a between the first and second jaw members 310, 320. The clip slot 314b of the first jaw member 310 is in vertical registration with the clip slot 324b of the second jaw member 320 to support the clip 340b between the first and second jaw members 310, 320.

The first sections 316a, 326a of respective tissue-contacting surfaces 316, 326 are electrically coupled together to collectively act as a first electrode (e.g., positively charged), and the second sections 316b, 326b of respective tissue-contacting surfaces 316, 326 are electrically coupled to collectively act as a second electrode (e.g., negatively charged) such that electrical energy conducted laterally across the first and second jaw members 310, 320. For example, the first and second sections 316a, 316b of the tissue-contacting surface 316 are configured to conduct electrical energy laterally across the knife channel 312 between the first and second sections 316a, 316b to seal a top surface of tissue "T" that is in contact with the tissue-contacting surface 316 toward the bottom surface of the tissue "T." Simultaneously, the first and second sections 326a, 326b of the tissue-contacting surface 326 are configured to conduct electrical energy laterally across the knife channel 322 between the first and second sections 326a, 326b to seal a bottom surface of tissue "T" that is in contact with the tissue-contacting surface 326 toward the top surface of the tissue "T."

In use, the movable handle 34 is actuated to enable the first and second jaw members 310, 320 to move from the open position (FIG. 5A) to the closed position (FIG. 5B) to thereby grasp tissue "T" disposed between the first and second jaw members 310, 320 while deforming the clips 340a, 340b onto the tissue "T" disposed between the first and second jaw members 310, 320. As the first and second jaw members 310, 320 move toward the closed position, the first and second jaw members 310, 320 simultaneously deform clips 340a, 340b about the tissue "T" grasped between the first and second jaw members 310, 320 similar to that described above with respect to the clips 140a, 140b.

As seen in FIGS. 5B and 5C, a clinician can then actuate electrical switch 50 to conduct electrosurgical energy laterally across the tissue-contacting surfaces 316, 326, which act as counterpart electrodes (e.g., anode-cathode), to seal the tissue "T" and create sealed tissue "S." With the clips 340a, 340b secured to the sealed tissue "S," the trigger 82 of the trigger assembly 80 (see FIG. 1) can be actuated to advance the knife blade 132 of the knife assembly 130 through the knife channels 312, 322 to sever the sealed tissue "S." The clips 340a, 340b reinforce the sealed tissue "S" similar to that described above with respect to clips 140a, 140b.

Any of the presently disclosed clips can be formed of any suitable biocompatible material. In some embodiments, any of the presently disclosed clips, or portions thereof, may be formed of a dielectric material. In certain embodiments, any of the presently disclosed clips, or portions thereof, may be formed of an electrically conductive material, whereby any of the presently disclosed clips may be utilized as an electrical conduit (e.g., via any of the presently described tissue-contacting surfaces) for conducting electrically energy directly and/or indirectly to tissue "T" to facilitate sealing of the tissue "T."

Any of the presently described jaw members, or portions thereof, may be made include dielectric or insulative material such as plastic (e.g., thermoplastic, thermosetting plastic, etc.), ceramic material, etc.

Any of the presently described jaw members, clips, or portions thereof, may be configured to conduct electrosurgical energy in any suitable fashion such as from clip to clip, from sealplate to sealplate, from sealplate to clip, and/or from clip to sealplate.

In some embodiments, one or more insulative or non-conductive stop members (not shown) may be included on any of the presently described tissue-contacting surfaces. Such stop members may function to provide a specific gap distance between first and second jaw members while in a closed position and/or may be configured to prevent tissue-contacting surfaces from contacting one another during the transmission of electrical energy.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

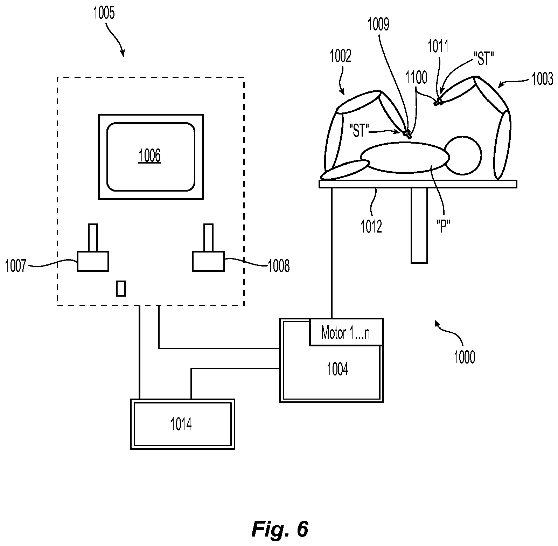

Referring also to FIG. 6, a medical work station is shown generally as work station 1000 and generally may include a plurality of robot arms 1002, 1003; a control device 1004; and an operating console 1005 coupled with the control device 1004. The operating console 1005 may include a display device 1006, which may be set up in particular to display three-dimensional images; and manual input devices 1007, 1008, by means of which a person (not shown), for example a clinician, may be able to telemanipulate the robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100 (e.g., a pair of jaw members), in accordance with any one of several embodiments disclosed herein, as will be described in greater detail below.

The robot arms 1002, 1003 may be driven by electric drives (not shown) that are connected to the control device 1004. The control device 1004 (e.g., a computer) may be set up to activate the drives, in particular by means of a computer program, in such a way that the robot arms 1002, 1003, their attaching devices 1009, 1011 and thus the surgical tool (including the end effector 1100) execute a desired movement according to a movement defined by means of the manual input devices 1007, 1008. The control device 1004 may also be set up in such a way that it regulates the movement of the robot arms 1002, 1003 and/or of the drives.

The medical work station 1000 may be configured for use on a patient "P" lying on a patient table 1012 to be treated in a minimally invasive manner by means of the end effector 1100. The medical work station 1000 may also include more than two robot arms 1002, 1003, the additional robot arms likewise connected to the control device 1004 and telemanipulatable by means of the operating console 1005. A medical instrument or surgical tool (including an end effector 1100) may also be attached to the additional robot arm. The medical work station 1000 may include a database 1014 coupled with the control device 1004. In some embodiments, preoperative data from patient/living being "P" and/or anatomical atlases may be stored in the database 1014.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. An end effector assembly, comprising:
   a first jaw member having a first tissue-contacting surface;
   a second jaw member coupled to the first jaw member and having a second tissue-contacting surface, the first and second tissue-contacting surfaces configured to conduct electrical energy to seal tissue disposed between the first and second jaw members; and
   at least one clip supported by the first and second jaw members, the first and second jaw members configured to deform the at least one clip such that the at least one clip reinforces a tissue seal formed between the first and second jaw members.
2. The end effector assembly of paragraph 1, further comprising a knife assembly including a knife blade configured to advance along at least one of the first and second jaw members to sever tissue sealed and clipped between the first and second jaw members.
3. The end effector assembly of paragraph 2, wherein at least one of the first and second jaw members includes a knife channel defined therein that extends along a length thereof and is configured to receive the knife blade of the knife assembly during translation thereof.
4. The end effector assembly of paragraph 3, wherein the first tissue-contacting surface includes a first section on a first side of the knife channel and a second section on a second side of the knife channel, wherein the first and second sections of the first tissue-contacting surface act as counterpart electrodes that conduct electrical energy laterally across the knife channel.
5. The end effector assembly of paragraph 4, wherein the second tissue-contacting surface includes a first section on the first side of the knife channel and a second section on the second side of the knife channel, wherein the first and second sections of the second tissue-contacting surface act as counterpart electrodes that conduct electrical energy laterally across the knife channel.
6. The end effector assembly of paragraph 1, wherein the at least one clip includes two clips supported between the first and second jaw members, the first and second jaw members positionable between open and closed positions to deform the two clips between the first and second jaw members.
7. The end effector assembly of paragraph 1, wherein at least one of the first and second jaw members defines at least one clip slot configured to support the at least one clip between the first and second jaw members.
8. The end effector assembly of paragraph 7, wherein the at least one clip slot includes a pair of clip slots defined on opposite sides of a knife channel defined in at least one of the first and second jaw members, the knife channel extending along a length of at least one of the first and second jaw members, and wherein the at least one clip includes a pair of clips, the pair of clip slots configured to support the pair of clips between the first and second jaw members.
9. The end effector assembly of paragraph 8, wherein the first and second tissue-contacting surfaces include seal plates that extend between the pair of clip slots, wherein the pair of clip slots electrically isolate the seal plates.
10. The end effector assembly of paragraph 9, wherein the seal plate of the first tissue-contacting surface acts as a first electrode and the seal plate of the second tissue-contacting surface acts as a second electrode, wherein the first and second electrodes conduct electrical energy between the pair of clip slots to seal tissue disposed between the pair of clip slots.
11. A forceps, comprising:
   a handle,
   an elongated shaft extending from the handle; and
   an end effector assembly secured to a distal end of the elongated shaft, the end effector assembly including:
      a first jaw member having a first tissue-contacting surface;
      a second jaw member coupled to the first jaw member and having a second tissue-contacting surface, the first and second tissue-contacting surfaces configured to conduct electrical energy to seal tissue disposed between the first and second jaw members; and
      at least one clip supported by the first and second jaw members, the first and second jaw members configured to deform the at least one clip such that the at least one clip reinforces a tissue seal formed between the first and second jaw members.
12. The forceps of paragraph 11, further comprising a knife assembly including a knife blade configured to advance along at least one of the first and second jaw members to sever tissue sealed and clipped between the first and second jaw members.
13. The forceps of paragraph 12, wherein at least one of the first and second jaw members includes a knife channel defined therein that extends long a length thereof and is configured to receive the knife blade of the knife assembly during translation thereof.
14. The forceps of paragraph 13, wherein the first tissue-contacting surface includes a first section on a first side of the knife channel and a second section on a second side of the knife channel, wherein the first and second sections of the first tissue-contacting surface act as counterpart electrodes that conduct electrical energy laterally across the knife channel.
15. The forceps of paragraph 14, wherein the second tissue-contacting surface includes a first section on the first side of the knife channel and a second section on the second side of the knife channel, wherein the first and second sections of the second tissue-contacting surface act as counterpart electrodes that conduct electrical energy laterally across the knife channel.
16. The forceps of paragraph 11, wherein the at least one clip includes two clips supported between the first and second jaw members, the first and second jaw members positionable between open and closed positions to deform the two clips between the first and second jaw members.
17. The forceps of paragraph 11, wherein at least one of the first and second jaw members defines at least one clip slot configured to support the at least one clip between the first and second jaw members.
18. The forceps of paragraph 17, wherein the at least one clip slot includes a pair of clip slots defined on opposite sides of a knife channel defined in at least one of the first and second jaw members, the knife channel extending along a length of at least one of the first and second jaw members, and wherein the at least one clip includes a pair of clips, the pair of clip slots configured to support the pair of clips between the first and second jaw members.
19. The forceps of paragraph 18, wherein the first and second tissue-contacting surfaces include seal plates that extend between the pair of clip slots, wherein the pair of clip slots electrically isolate the seal plates.
20. The forceps of paragraph 19, wherein the seal plate of the first tissue-contacting surface acts as a first electrode and the seal plate of the second tissue-contacting surface acts as a second electrode, wherein the first and second electrodes conduct electrical energy between the pair of clip slots to seal tissue disposed between the pair of clip slots.

## Claims

1. An end effector assembly, comprising:
a first jaw member having a first tissue-contacting surface;
a second jaw member coupled to the first jaw member and having a second tissue-contacting surface, the first and second tissue-contacting surfaces configured to conduct electrical energy to seal tissue disposed between the first and second jaw members; and
at least one clip supported by the first and second jaw members, the first and second jaw members configured to deform the at least one clip such that the at least one clip reinforces a tissue seal formed between the first and second jaw members.

2. The end effector assembly of claim 1, further comprising a knife assembly including a knife blade configured to advance along at least one of the first and second jaw members to sever tissue sealed and clipped between the first and second jaw members.

3. The end effector assembly of claim 2, wherein at least one of the first and second jaw members includes a knife channel defined therein that extends along a length thereof and is configured to receive the knife blade of the knife assembly during translation thereof.

4. The end effector assembly of claim 3, wherein the first tissue-contacting surface includes a first section on a first side of the knife channel and a second section on a second side of the knife channel, wherein the first and second sections of the first tissue-contacting surface act as counterpart electrodes that conduct electrical energy laterally across the knife channel.

5. The end effector assembly of claim 4, wherein the second tissue-contacting surface includes a first section on the first side of the knife channel and a second section on the second side of the knife channel, wherein the first and second sections of the second tissue-contacting surface act as counterpart electrodes that conduct electrical energy laterally across the knife channel.

6. The end effector assembly of any preceding claim, wherein at least one of the first and second jaw members defines at least one clip slot configured to support the at least one clip between the first and second jaw members.

7. The end effector assembly of claim 6, wherein the at least one clip slot includes a pair of clip slots defined on opposite sides of a knife channel defined in at least one of the first and second jaw members, the knife channel extending along a length of at least one of the first and second jaw members, and wherein the at least one clip includes a pair of clips, the pair of clip slots configured to support the pair of clips between the first and second jaw members.

8. The end effector assembly of claim7, wherein the first and second tissue-contacting surfaces include seal plates that extend between the pair of clip slots, wherein the pair of clip slots electrically isolate the seal plates, preferably wherein the seal plate of the first tissue-contacting surface acts as a first electrode and the seal plate of the second tissue-contacting surface acts as a second electrode, wherein the first and second electrodes conduct electrical energy between the pair of clip slots to seal tissue disposed between the pair of clip slots.

9. A forceps, comprising:
a handle,
an elongated shaft extending from the handle; and
an end effector assembly secured to a distal end of the elongated shaft, the end effector assembly including:
a first jaw member having a first tissue-contacting surface;
a second jaw member coupled to the first jaw member and having a second tissue-contacting surface, the first and second tissue-contacting surfaces configured to conduct electrical energy to seal tissue disposed between the first and second jaw members; and
at least one clip supported by the first and second jaw members, the first and second jaw members configured to deform the at least one clip such that the at least one clip reinforces a tissue seal formed between the first and second jaw members.

10. The forceps of claim 9, further comprising a knife assembly including a knife blade configured to advance along at least one of the first and second jaw members to sever tissue sealed and clipped between the first and second jaw members.

11. The forceps of claim 10, wherein at least one of the first and second jaw members includes a knife channel defined therein that extends long a length thereof and is configured to receive the knife blade of the knife assembly during translation thereof.

12. The forceps of claim 11, wherein the first tissue-contacting surface includes a first section on a first side of the knife channel and a second section on a second side of the knife channel, wherein the first and second sections of the first tissue-contacting surface act as counterpart electrodes that conduct electrical energy laterally across the knife channel.

13. The forceps of claim 12, wherein the second tissue-contacting surface includes a first section on the first side of the knife channel and a second section on the second side of the knife channel, wherein the first and second sections of the second tissue-contacting surface act as counterpart electrodes that conduct electrical energy laterally across the knife channel.

14. The forceps of any preceding claim, wherein at least one of the first and second jaw members defines at least one clip slot configured to support the at least one clip between the first and second jaw members.

15. The forceps of claim 14, wherein the at least one clip slot includes a pair of clip slots defined on opposite sides of a knife channel defined in at least one of the first and second jaw members, the knife channel extending along a length of at least one of the first and second jaw members, and wherein the at least one clip includes a pair of clips, the pair of clip slots configured to support the pair of clips between the first and second jaw members, preferably , wherein the first and second tissue-contacting surfaces include seal plates that extend between the pair of clip slots, wherein the pair of clip slots electrically isolate the seal plates , and preferably wherein the seal plate of the first tissue-contacting surface acts as a first electrode and the seal plate of the second tissue-contacting surface acts as a second electrode, wherein the first and second electrodes conduct electrical energy between the pair of clip slots to seal tissue disposed between the pair of clip slots.
